(19) Europäisches Patentamt
European Patent Office
Office européen des brevets

(11) Numéro de publication: **0 428 438 A1**

(12) **DEMANDE DE BREVET EUROPEEN**

(21) Numéro de dépôt: **90403155.6**

(51) Int. Cl.5: **A61K 31/445**

(22) Date de dépôt: **07.11.90**

(30) Priorité: **14.11.89 FR 8914868**

(43) Date de publication de la demande:
**22.05.91 Bulletin 91/21**

(84) Etats contractants désignés:
**AT BE CH DE DK FR GB IT LI LU NL SE**

(71) Demandeur: **SYNTHELABO**
**58 rue de la Glacière**
**F-75013 Paris(FR)**

(72) Inventeur: **Langer, Salomon**
**92, rue Jouffroy**
**F-75017 Paris(FR)**
Inventeur: **Frost, Jonathan**
**23, route de Montjean**
**F-91320 Wissous(FR)**
Inventeur: **Schoemaker, Johannes**
**29, Allée des Graviers de la Salmouille**
**F-91190 Gif Sur Yvette(FR)**
Inventeur: **Gaudilliere,Bernard**
**28, rue Zilina**
**F-92000 Nanterre(FR)**
Inventeur: **Bertin, Jean**
**55, rue d'Estienne d'Orves**
**F-92140 Clamart(FR)**
Inventeur: **Rousseau, Jean**
**17bis, Avenue de Montrouge**
**F-92340 Bourg La Reine(FR)**
Inventeur: **Dupont, Régis**
**152 Quai Paul Bert**
**F-37100 Tours(FR)**
Inventeur: **Wick, Alexander**
**10, Boulevard des Plants**
**F-78860 St-Nom-la-Breteche(FR)**

(74) Mandataire: **Ludwig, Jacques et al**
**SYNTHELABO, Service Brevets, 22, avenue Galilée, B.P. 72**
**F-92352 Le Plessis Robinson Cédex(FR)**

(54) **Utilisation d'ifenprodil et de ses dérivés pour la fabrication de médicaments antipsychotiques.**

(57) Utilisation d'Ifenprodil (α-(4-Hydroxyphenyl)-β-methyl-4-(phenylmethyl)-1-piperidineethanol) et de ses dérivés pour la fabrication de médicaments antipsychotiques.
De tels médicaments sont utilisables pour le traitement de psychoses telles que la schizophrénie.

EP 0 428 438 A1

# UTILISATION D'IFENPRODIL ET DE SES DERIVES POUR LA FABRICATION DE MEDICAMENTS ANTIPSYCHOTIQUES

La présente invention a pour objet l'utilisation d'Ifenprodil et de ses dérivés, de formule générale (I), pour la fabrication de médicaments antipsychotiques

Dans la formule générale (I), donnée plus loin, R1 désigne un atome d'halogène ou un groupe hydroxy, R2 désigne un atome d'hydrogène ou un groupe méthyle, et R3 désigne un atome d'hydrogène ou d'halogène.

Selon que R2 désigne un atome d'hydrogène ou un groupe méthyle, la molécule d'un composé de formule générale (I) comporte un ou deux atomes de carbone asymétriques. Les composés utilisables selon l'invention peuvent donc exister sous diverses formes stéréochimiques. Ils peuvent également se présenter à l'état de bases libres ou de sels d'addition des acides.

Les composés utilisables selon l'invention sont décrits dans les brevets US-3509164, FR-2546166 et EP-0109317, et dans C.A., 94, 83708b (1981).

Les composés de formule générale (I) ont fait l'objet d'essais pharmacalogiques qui ont mis en évidence leurs propriétés antipsychotiques.

Ainsi, en particulier, ils ont été soumis à un essai d'inhibition de la liaison de la $[^3H]$-(+)-3-(3-hydroxyphényl)-N-(1-propyl)pipéridine, ou $[^3H]$-(+)-3-PPP, aux récepteurs $\sigma$ du cerveau de rat, d'après le protocole décrit par Largent et coll., dans J. Pharmacol. Exp. Ther., 238, 739-748 (1986). On sacrifie des rats mâles Sprague-Dawley de 150 à 200 g, et on en homogénéise le cortex cérébral dans 25 volumes de tampon Tris-HCl à 50 mM (pH = 7,4 à 25 °C) glacé, au moyen d'un appareil Ultra-Turrax™. On lave le mélange deux fois en le centrifugeant pendant 10 mn à 45000g et en remettant le culot en suspension dans du tampon frais. On dilue le culot lavé dans 20 volumes de tampon Tris-HCl à 50 mM (pH = 8,0 à 22 °C), et on fait incuber des fractions aliquotes de 75 µl dans un volume final de 250 µl à 2 nM de $[^3H]$-(+)-3-PPP (activité spécifique : 90 Ci/mmole, d'origine New England Nuclear), pendant 90 mn à 25 °C, en l'absence ou en présence de substances compétitrices.

Après incubation on récupère les membranes par filtration sur des filtres Whatman GF/B™ traités avec de la polyéthylèneimine à 0,05%, au moyen d'un appareil Skatron Cell Harvester™, et on les lave avec environ 2,5 ml de tampon Tris-HCl (pH = 7,7 à 0 °C) glacé.

On détermine la liaison non spécifique avec du halopéridol 1µM, on analyse les données selon les méthodes usuelles, et on calcule pour chaque composé la concentration $IC_{50}$, qui inhibe de 50% la liaison du $[^3H]$-(+)-3PPP.

Le tableau suivant indique les $IC_{50}$ (en PM) pour quelques composés utilisables selon l'invention.

Tableau

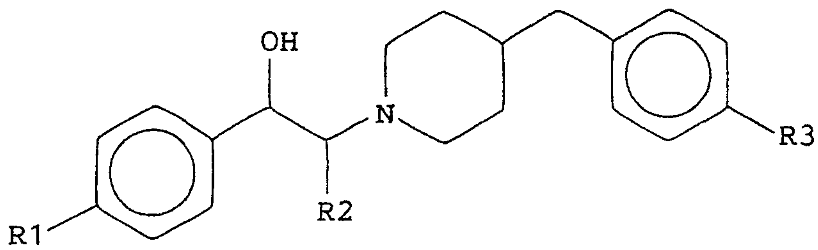

(I)

| N° | R1 | R2 | R3 | Forme | Sel/ base | $IC_{50}$ |
|---|---|---|---|---|---|---|
| 1 | OH | $CH_3$ | H | (±)érythro | HCl | 0,008 |
| 2 | OH | $CH_3$ | H | (±)thréo | base | 0,01 |
| 3 | OH | $CH_3$ | H | (-)érythro | Bz. | 0,015 |
| 4 | OH | $CH_3$ | H | (+)érythro | Bz. | 0,007 |
| 5 | Cl | H | F | (±) | HCl | 0,003 |
| 6 | Cl | H | F | (+) | base | 0,0092 |
| 7 | Cl | H | F | (-) | base | 0,0098 |

Note : dans la colonne Sel/base, "Bz." désigne un benzoate et "HCl" désigne un chlorhydrate.

Les résultats des essais pharmacologiques montrent que les composés utilisables selon l'invention sont susceptibles d'avoir une activité antipsychotique chez l'homme, et qu'ils sont donc utilisables pour le traitement des psychoses, en particulier de la schizophrénie.

Ils peuvent être présentés sous toutes formes galéniques, associés à des excipients appropriés, pour l'administration entérale ou parentérale, par exemple sous forme de comprimés, dragées, gélules, solutions ou suspensions buvables ou injectables, suppositoires, etc, dosés pour permettre une dose journalière de 1 à 120 mg de substance active.

**Revendications**

1. Utilisation d'un composé de formule générale I

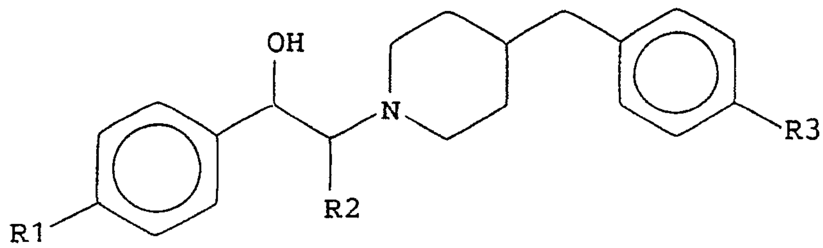

(I)

dans laquelle R1 désigne un atome d'halogène ou un groupe hydroxy, R2 désigne un atome d'hydrogène ou un groupe méthyle, et R3 désigne un atome d'hydrogène ou d'halogène, sous forme d'isomère optique pur ou de mélange de tels isomères, à l'état de base libre ou de sel d'addition à un acide acceptable en pharmacologie, pour la fabrication d'un médicament antipsychotique.

2. Utilisation selon la revendication 1, caractérisée en ce que le composé de formule générale (I) est le (±)-érythro-α-(4-hydroxyphényl)-β-méthyl-4-(phénylméthyl)-1-pipéridineéthanol.

3. Utilisation selon la revendication 1, caractérisée en ce que le composé de formule générale (I) est le (±)-thréo-α-(4-hydroxyphényl)-β-méthyl-4-(phénylméthyl)-1-pipéridineéthanol.

4. Utilisation selon la revendication 1, caractérisée en ce que le composé de formule générale (I) est le (±)-érythro-α-(4-hydroxyphényl)-β-méthyl-4-(phénylméthyl)-1-pipéridineéthanol.

5. Utilisation selon la revendication 1, caractérisée en ce que le composé de formule générale (I) est le (-)érythro-α-(4-hydroxyphényl)-β-méthyl-4-(phénylméthyl)-1-pipéridineéthanol.

6. Utilisation selon la revendication 1, caractérisée en ce que le composé de formule générale (I) est le (±)-α-(4-chlorophényl)-4-[(4-fluorophényl)méthyl]-1-pipéridineéthanol.

7. Utilisation selon la revendication 1, caractérisée en ce que le composé de formule générale (I) est le (+)-α-(4-chlorophényl)-4-[(4-fluorophényl)méthyl]-1-pipéridineéthanol.

8. Utilisation selon la revendication 1, caractérisée en ce que le composé de formule générale (I) est le (-)-α-(4-chlorophényl)-4-[(4-fluorophényl)méthyl]-1-pipéridineéthanol.

Office européen des brevets

# RAPPORT DE RECHERCHE EUROPEENNE

Numéro de la demande

**EP 90 40 3155**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | PSYCHOLOGIE MEDICALE, vol. 13, no. 9, 1981, pages 1483-1489, S.P.E.I., Paris, FR; C. FERRIERE et al.: "Etude clinique du tartrate d'ifenprodil en milieu neuro-psychiatrique" * Résumé; page 1484, "nature de l'echantilion"; page 1486, "conclusions"; page 1487, references no. 9,16 * | 1-8 | A 61 K 31/445 |
| A,D | EP-A-0 109 317 (SYNTHELABO) * Revendications 1-7,13; pages 54-55 * | 1-8 | |
| A | EUROPEAN JOURNAL OF PHARMACOLOGY, vol. 162, 1989, pages 157-166, Elsevier Science Publishers B.V. (Biomedical Division); A. SERRANO et al.: "NMDA antagonists block restraint-induced increase in extracellular DOPAC in rat nucleus accumbens" * Résumé; pages 161-164: "discussion" * | 1-8 | |
| A | R. BERKOW: "The Merck Manual", edition 15, 1987, chapitre 281, pages 2479-2492, Merck Sharp & Dohme Research Laboratories; "Drug acting on the central nervous system" * Page 2483 "Antianxiety agents" - page 2492 * | 1-8 | **DOMAINES TECHNIQUES RECHERCHES (Int. Cl.5)** |
| P,X | EUROPEAN JOURNAL OF PHARMACOLOGY, vol. 176, 1990, pages 247-248, Elsevier Science Publishers B.V. (Biomedical Division); E.W. KARBON et al.: "Ifenprodil potently interacts with [3H](+)-3-PPP-labeled gamma binding sites in guinea pig brain membranes" * En entier * | 1-8 | A 61 K |

Le présent rapport de recherche a été établi pour toutes les revendications

| Lieu de la recherche | Date d'achèvement de la recherche | Examinateur |
|---|---|---|
| La Haye | 24 janvier 91 | ISERT B. |

CATEGORIE DES DOCUMENTS CITES

X: particulièrement pertinent à lui seul
Y: particulièrement pertinent en combinaison avec un autre document de la même catégorie
A: arrière-plan technologique
O: divulgation non-écrite
P: document intercalaire
T: théorie ou principe à la base de l'invention
E: document de brevet antérieur, mais publié à la date de dépôt ou après cette date
D: cité dans la demande
L: cité pour d'autres raisons
&: membre de la même famille, document correspondant